# DEMANDE DE BREVET EUROPEEN

(11) **EP 1 473 021 A1**
(43) Date de publication de la demande: **03.11.2004**
(21) Numéro de dépôt: 04291079.4
(22) Date de dépôt: 26.04.2004
(51) Int. Cl.: A61K 7/06, A61K 7/50

(54) **Utilisation de nitrure de bore associé à au moins une huile pour le conditionnement des cheveux et composition cosmétique les comprenant**

(30) Priorité: 28.04.2003 FR 0305184
(71) Demandeur: L'OREAL, 75008 Paris (FR)
(72) Inventeur: Parris, Eric, 92600 Asnières (FR); Gawtrey, Jonathan, 92100 Boulogne (FR)
(74) Mandataire: Casalonga, Axel

(57) **Abrégé**

La présente invention décrit une utilisation de nitrure de bore associé à au moins une huile pour le conditionnement des cheveux et une composition cosmétique comprenant, dans un milieu aqueux cosmétiquement acceptable, du nitrure de bore associé à au moins une huile en un rapport pondéral nitrure de bore/huile(s) supérieur ou égal à 50/50.

## Description

La présente invention concerne une utilisation du nitrure de bore associé à des huiles dans des compositions de traitement capillaire, une composition de traitement capillaire contenant du nitrure de bore associé à des huiles, et un procédé de traitement capillaire mettant en oeuvre cette composition.

Dans le domaine de la cosmétique, on cherche notamment à améliorer le conditionnement des cheveux. Par conditionnement, on entend des propriétés de démêlage facile, de brillance, de douceur au toucher et de glissant.

L'utilisation de particules de nitrure de bore est connue en cosmétique, et plus particulièrement dans le domaine du maquillage ou dans des compositions de protection contre le rayonnement ultraviolet comme cela est décrit dans les demandes de brevet DE 199 23 773 et DE 199 23 667.

La demande EP 1 077 058 décrit des compositions cosmétiques ou dermatologiques, de type eau-dans-huile, sans émulsifiant comprenant du nitrure de bore qui peut être enrobé avec un polydiméthylsiloxane, dans des compositions de soin de la peau.

La Demanderesse a trouvé de manière surprenante qu'en utilisant, dans des compositions de traitement capillaire, des particules de nitrure de bore en association avec au moins une huile, il était possible d'obtenir un très bon effet de conditionnement des cheveux. Cet effet de conditionnement est encore amélioré en utilisant un certain rapport pondéral nitrure de bore/huile(s).

Sans être tenu par une quelconque théorie, il semblerait que dans ces conditions, on augmente significativement le dépôt de nitrure de bore sur les cheveux, d'où une efficacité accrue.

Par ailleurs, cet effet de conditionnement peut être rémanent au rinçage et au passage du peigne ou d'une brosse à cheveux.

L'invention a donc pour objet une utilisation du nitrure de bore associé à au moins une huile pour le conditionnement des cheveux, et notamment dans les compositions de traitement capillaire, et par exemple, dans des shampoings ou des après-shampoings.

Un autre objet de la présente invention est une composition cosmétique comprenant, dans un milieu aqueux cosmétiquement acceptable, du nitrure de bore associé à au moins une huile, en un rapport nitrure de bore/huile(s) particulier.

Un objet supplémentaire de la présente invention est un procédé de traitement des matières kératiniques mettant en oeuvre une composition cosmétique selon l'invention.

D'autres objets, caractéristiques, aspects et avantages de l'invention apparaîtront encore plus clairement à la lecture de la description et des divers exemples qui suivent.

Selon l'invention, on utilise du nitrure de bore associé à au moins une huile dans une compositions cosmétique, pour le conditionnement des cheveux.

Le nitrure de bore utilisé dans la présente invention peut être enrobé par cette huile ou ces huiles. L'enrobage est effectué, de préférence, avant introduction dans la composition de traitement capillaire via toutes les technologies classiques telles que, par exemple, extrusion ou addition d'un tiers solvant volatil.

Le rapport pondéral nitrure de bore/huile(s) est de préférence supérieur ou égal à 50/50, mieux encore supérieur ou égal à 60/40, plus préférentiellement compris entre 60/40 et 90/10, et encore plus préférentiellement compris entre 80/20 et 90/10.

Le nitrure de bore présente de préférence une taille primaire moyenne en nombre comprise entre 1 et 50 *µ*m, mieux encore entre 5 et 30 µm, et plus préférentiellement entre 5 et 25 *µ*m, et encore plus préférentiellement compris entre 7 et 15 *µ*m.

Au sens de la présente invention, on entend par "taille primaire de particule", la dimension maximale qu'il est possible de mesurer entre deux points diamétralement opposés d'une particule individuelle. La taille peut être déterminée, par exemple, par microscopie électronique à transmission ou à partir de la mesure de la surface spécifique par la méthode BET ou bien par l'intermédiaire d'un granulomètre laser.

Les particules selon l'invention peuvent avoir une forme quelconque, par exemple, de préférence la forme de lamelles, de paillettes, d'aiguilles, de plaquettes ou des formes totalement aléatoires.

Le nitrure de bore tel que défini ci-dessus est de préférence utilisé en une quantité comprise entre 0,01 et 20 % en poids, encore plus préférentiellement en une quantité comprise entre 0,1 et 10 % en poids par rapport au poids de la composition.

Par huile, on entend au sens de la présente invention, une substance liquide à température ambiante, par exemple de 25 °C, et sous pression atmosphérique, et insoluble dans l'eau.

Par insoluble dans l'eau, on entend au sens de la présente invention, une substance qui présente une solubilité dan l'eau pure inférieure à 1 % à 25 °C et sous pression atmosphérique.

Les huiles utilisées dans la présente invention présente de préférence une viscosité dynamique à 25 °C, inférieure à 1 Pa.s (1000 cps), de préférence comprise entre 10⁻³ et 0,1 Pa.s (1 et 100 cps). La viscosité dynamique est mesurée à 25 °C avec un taux de cisaillement de 100 s⁻¹, par exemple, avec l'appareil référencé RM 180 Rheomat de la société METTLER.

Les huiles pouvant être utilisées dans la présente invention sont choisies de préférence, parmi les huiles végétales, les huiles minérales, les huiles de synthèse, les huiles siliconées et les esters gras.

Parmi les huiles végétales pouvant être utilisées dans la présente invention, on peut notamment citer l'huile d'amande douce, l'huile d'avocat, l'huile de ricin, l'huile d'olive, l'huile de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, le beurre de karité, l'huile de palme, l'huile de noyau d'abricot et l'huile de calophyllum.

Des exemples d'huiles minérales sont l'huile de paraffine et l'huile de vaseline.

Les huiles de synthèse peuvent notamment être choisies parmi les polydécènes, le squalane, les poly(α-oléfines) comme l'isododécane ou l'isohexadécane, les huiles végétales transestérifiées et les huiles fluorées.

On peut également utiliser des esters d'acide gras, tels que par exemple, les composés de formule RₐCOOR_{b} dans laquelle Rₐ représente le reste d'un acide gras supérieur comportant de 6 à 29 atomes de carbone et R_{b} représente une chaîne hydrocarbonée contenant de 3 à 30 atomes de carbone, telles que l'huile de Purcellin (octanoate de stéaryle), le myristate d'isopropyle, le palmitate d'isopropyle, le stéarate de butyle, le laurate d'hexyle, l'adipate de diisopropyle, l'isononanoate d'isononyle, le palmitate de 2-éthylhexyle, le laurate de 2-hexyldécyle, le palmitate de 2-octyldécyle, le myristate ou le lactate de 2-octyldodécyle.

A titre d'exemples d'huiles siliconées, on peut notamment citer les polydiméthylsiloxanes (PDMS), les polyorganosiloxanes phénylés tels que les phényltriméthicones, les phényltriméthylsiloxydiphénylsiloxanes, les diphénylméthyldiméthyltrisiloxanes, les diphényldiméthicones, les phényldiméthicones, les polyméthyl-phénylsiloxanes, éventuellement fluorés ; les polysiloxanes modifiés par des acides gras, des alcools gras ou des polyoxyalkylènes, les silicones fluorées, les huiles siliconées perfluorées.

Parmi les huiles siliconées préférées, on peut citer les polydiméthylsiloxanes, les polyméthylphénylsiloxanes, les silicones comportant des séquences ou des greffons polyoxyalkylènes, en particulier polyoxyéthylène ou copoly(oxyéthylène/oxypropylène) telles que les diméthiconecopolyols, les silicones portant à la fois des groupes hydrophobes hydrocarbonés (par exemple des groupes alkyle en C₂-C₃₀) et des séquences ou greffons polyoxyéthylénés ou copoly(oxyéthylénés/oxypropylénés) telles que les alkyldiméthiconecopolyols, les silicones portant des groupes fluorés ou perfluorés telles que les polydiméthylsiloxanes perfluoroalkylés et les polyméthylphénylsiloxanes perfluoroalkylés, et leurs mélanges.

Ces huiles siliconées peuvent comporter éventuellement des groupes alkyle ou alcoxy en bout de chaîne siliconée ou pendante.

Comme huile siliconée utilisable dans l'invention, on peut encore citer les silicones linéaires ou cycliques, et comportant en particulier de 2 à 7 atomes de silicium. On peut notamment citer l'octaméthylcyclotétrasiloxane, le décaméthylcyclopentasiloxane, l'hexadécaméthylcyclohexasiloxane, l'heptaméthylhexyltrisiloxane, l'heptaméthyloctyltrisiloxane et leurs mélanges.

Les huiles particulièrement préférées dans le cadre de la présente invention sont l'huile d'avocat, l'isohexadécane, une paraffine liquide et les polydiméthylsiloxanes.

La ou les huile(s) telle(s) que définie(s) ci-dessus est ou sont de préférence utilisée(s) en une quantité comprise entre 0,01 et 20 % en poids, encore plus préférentiellement en une quantité comprise entre 0,1 et 10 % en poids par rapport au poids de la composition.

Le nitrure de bore associé à au moins une huile, tels que décrits ci-dessus, peuvent être utilisés dans une composition cosmétique telle que, par exemple, dans un shampoing, un après-shampoing, un produit de fixation et/ou de maintien de la coiffure.

La présente invention a encore pour objet une composition cosmétique comprenant, dans un milieu aqueux cosmétiquement acceptable, du nitrure de bore et au moins une huile en un rapport pondéral nitrure de bore/huile supérieur ou égal à 50/50, de préférence supérieur ou égal à 60/40, plus préférentiellement compris entre 60/40 et 90/10, et encore plus préférentiellement compris entre 80/20 et 90/10.

Le nitrure de bore tel que défini ci-dessus est de préférence présent en une quantité comprise entre 0,01 et 20 % en poids, encore plus préférentiellement en une quantité comprise entre 0,1 et 10 % en poids par rapport au poids de la composition.

Les huiles des compositions de l'invention peuvent être choisies parmi celles décrites ci-dessus, et sont de préférence non siliconées.

La ou les huile(s) telle(s) que définie(s) ci-dessus est ou sont de préférence présente(s) en une quantité comprise entre 0,01 et 20 % en poids, encore plus préférentiellement en une quantité comprise entre 0,1 et 10 % en poids par rapport au poids de la composition.

Par milieu cosmétiquement acceptable, on entend un milieu compatible avec les cheveux, mais aussi d'odeur, d'aspect et de toucher agréables.

Le milieu aqueux cosmétiquement acceptable comprend l'eau ou un mélange d'eau et d'un solvant cosmétiquement acceptable choisi parmi les alcools inférieurs en C₁-C₄, tels que l'éthanol, l'isopropanol, le tertio-butanol ou le n-butanol ; les polyols tels que le glycérol, le propylèneglycol et les polyéthylèneglycols ; et leurs mélanges.

L'eau est de préférence présente en une quantité allant de 50 à 99 % en poids, mieux encore de 60 à 95 % en poids et encore plus préférentiellement de 75 à 95 % en poids par rapport au poids total de la composition.

La composition selon l'invention peut comprendre en outre un ou plusieurs additifs classiques bien connus dans la technique, tels que des agents tensioactifs anioniques, cationiques, non ioniques, amphotères ou zwittérioniques ; des polymères anioniques, cationiques, non-ioniques amphotères ou zwitteroniques ; des filtres UV ; des parfums ; des agents nacrants ; des épaississants naturels ou synthétiques ; des alcools gras en C₁₂-C₃₀ ; des colorants ; des conservateurs ; des agents de stabilisation de pH ; des vitamines, des provitamines ; des agents antibactériens ; des agents anti-oxydants ou réducteurs, et des agents acides ou alcalins.

A titre de tensioactifs anioniques utilisables, seuls ou en mélanges, dans le cadre de la présente invention, on peut citer notamment les sels, en particulier les sels de métaux alcalins tels que les sels de sodium, les sels d'ammonium, les sels d'amines, les sels d'aminoalcools ou les sels de métaux alcalino-terreux, par exemple, de magnésium, des composés suivants : les sulfates d'alkyle, les alkyléther-sulfates, les alkylamidoéthersulfates, les alkylarylpolyéthersulfates, les monoglycéride-sulfates ; les alkylsulfonates, les phosphates d'alkyle, les alkylamidesulfonates, les alkylarylsulfonates, les α-oléfine-sulfonates, les paraffine-sulfonates ; les sulfosuccinates d'alkyle, les alkyléthersulfosuccinates, les alkylamidesulfosuccinates ; les sulfoacétates d'alkyle ; les acylsarcosinates ; et les acylglutamates, les groupes alkyle ou acyle de tous ces composés comportant de 6 à 24 atomes de carbone et le groupe aryle désignant de préférence un groupe phényle ou benzyle. On peut également utiliser les esters d'alkyle en C₆-C₂₄ et d'acides polyglycoside-carboxyliques tels que les glucoside-citrates d'alkyle, les polyglycoside-tartrates d'alkyle, et les polyglycoside-sulfosuccinates d'alkyle ; les sulfosuccinamates d'alkyle, les iséthionates d'acyle et les N-acyltaurates, le groupe alkyle ou acyle de tous ces composés comportant de 12 à 20 atomes de carbone. Parmi les tensioactifs anioniques encore utilisables, on peut également citer les lactylates d'acyle dont le groupe acyle comporte de 8 à 20 atomes de carbone.

En outre, on peut encore citer les acides d'alkyl-D-galactoside uroniques et leurs sels ainsi que les acides alkyl(C₆-C₂₄)éthercarboxyliques polyoxyalkylénés, les acides alkyl(C₆-C₂₄)aryl(C₆-C₂₄)éther-carboxyliques polyoxyalkylénés, les acides alkyl(C₆-C₂₄)amidoéther carboxyliques polyoxyalkylénés et leurs sels, en particulier ceux comportant de 2 à 50 groupements oxyde d'éthylène, et leurs mélanges.

Parmi les tensioactifs anioniques cités ci-dessus, on préfère utiliser selon l'invention les sels de sulfates d'alkyle, d'alkyléthersulfates, comme le lauryléthersulfate de sodium, et d'alkyléthercarboxylates, et leurs mélanges.

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178). Ainsi, ils peuvent être notamment choisis parmi les alcools, les alpha-diols, les alkyl(C₁-C₂₀)phénols ou les acides gras polyéthoxylés, polypropoxylés ou polyglycérolés, ayant une chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitane éthoxylés ayant de 2 à 30 moles d'oxyde d'éthylène ; les esters d'acides gras du saccharose, les esters d'acides gras du polyéthylèneglycol, les alkyl(C₆-C₂₄)polyglycosides, les dérivés de N-alkyl(C₆-C₂₄)glucamine, les oxydes d'amines tels que les oxydes d'alkyl(C₁₀-C₁₄)amines ou les oxydes de N-acyl(C₁₀-C₁₄)aminopropylmorpholine ; et leurs mélanges.

Parmi les tensioactifs non-ioniques cités ci-dessus, on utilise de préférence les alkyl(C₆-C₂₄)polyglycosides.

Les agents tensioactifs amphotères, convenant dans la présente invention, peuvent être notamment des dérivés d'amines aliphatiques secondaires ou tertiaires, dans lesquels le groupe aliphatique est une chaîne linéaire ou ramifiée comportant de 8 à 22 atomes de carbone et contenant, au moins un groupe anionique hydrosolubilisant tel que, par exemple, un groupe carboxylate, sulfonate, sulfate, phosphate ou phosphonate ; on peut citer encore les alkyl(C₈-C₂₀)bétaïnes, les sulfobétaïnes, les alkyl(C₈-C₂₀)amidoalkyl(C₆-C₈)-bétaïnes ou les alkyl(C₈-C₂₀)amidoalkyl(C₆-C₈)sulfobétaïnes ; et leurs mélanges.

Parmi les dérivés d'amines, on peut citer les produits commercialisés sous la dénomination MIRANOL® , tels que décrits dans les brevets US-2 528 378 et US-2 781 354 et classés dans le dictionnaire CTFA, 3^{ème} édition, 1982, sous les dénominations Amphocarboxyglycinate et Amphocarboxypropionate de structures respectives (2) et (3) :

R₂-CONHCH₂CH,₂-N⁺(R₃)(R₄)(CH₂COO⁻) (2)

dans laquelle :
R₂ représente un groupe alkyle dérivé d'un acide R₂-COOH présent dans l'huile de coprah hydrolysée, un groupe heptyle, nonyle ou undécyle,
R₃ représente un groupe bêta-hydroxyéthyle, et
R₄ représente un groupe carboxyméthyle ;
et

R_{2'}-CONHCH₂CH₂-N(B)(C) (3)

dans laquelle :
B représente -CH₂CH₂OX',
C représente -(CH₂)_{z}- Y', avec z = 1 ou 2,
X' représente le groupe -CH₂CH₂-COOH ou un atome d'hydrogène,
Y' représente -COOH ou le groupe -CH₂-CHOH-SO₃H,
R₂, représente un groupe alkyle d'un acide R_{g} -COOH présent dans l'huile de coprah ou dans l'huile de lin hydrolysée, un groupe alkyle, notamment en C₁₇ et sa forme iso, un groupe en C₁₇ insaturé.

Ces composés sont classés dans le dictionnaire CTFA, 5ème édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

A titre d'exemple, on peut citer le cocoamphodiacétate commercialisé sous la dénomination commerciale MIRANOL® C2M concentré par la société RHODIA.

Parmi les tensioactifs amphotères, on utilise de préférence les alkyl(C₈-C₂₀)bétaïnes, les alkyl(C₈-C₂₀)amidoalkyl(C₆-C₈)bétaïnes, les alkylamphodiacétates et leurs mélanges.

A titre de tensioactif cationique, on peut notamment citer cationiques les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkylammonium ou d'alkylpyridinium ; les dérivés d'imidazoline ; ou les oxydes d'amines à caractère cationique.

Les tensioactifs cationiques utilisés de préférence dans la présente invention sont les sels d'alkylamidoalkyltrialkylammonium, les dérivés d'imidazoline, ou les oxydes d'amines à caractère cationique.

L'homme de métier veillera à choisir les éventuels additifs et leur quantité de manière à ce qu'ils ne nuisent pas aux propriétés des compositions de la présente invention.

De préférence, la composition de l'invention est liquide, et encore plus préférentiellement se présente sous la forme d'une émulsion huile-dans-eau.

Les compositions conformes à l'invention peuvent être utilisées pour le traitement cosmétique des cheveux, comme shampoings, après-shampoings, produits de fixation et/ou de maintien de la coiffure.

Un autre objet de l'invention est un procédé de traitement capillaire des cheveux qui consiste à appliquer une quantité efficace d'une composition telle que décrite ci-dessus, sur les cheveux, à rincer ou non après un éventuel temps de pose.

Les exemples suivants illustrent la présente invention.

### EXEMPLES

### Exemple 1

On a préparé du nitrure de bore enrobé à partir des composants indiqués dans le tableau ci-dessous dans les rapports indiqués ci-dessous :

| Huile d'enrobage | Nitrure de bore | Rapport pondéral Nitrure de bore/huile |
|---|---|---|
| Huile d'avocat | Poudre de Nitrure de bore⁽⁴⁾, de taille moyenne des particules de 9-11 *µ*m | 80/20 |
| | | 90/10 |
| Isohexadécane⁽¹⁾ | | 80/20 |
| | | 75/25 |
| Diméthicone⁽²⁾ | | 80/20 |
| | | 75/25 |
| Paraffine liquide⁽³⁾ | | 80/20 |

| | | |
|---|---|---|
| ⁽¹⁾ vendu sous la dénomination commerciale Permethyl 101A par la société Bayer | | |
| ⁽²⁾ vendu sous la dénomination commerciale DC 200 Fluid par la société Dow Corning - 5 mm²/s | | |
| ⁽³⁾ vendu sous la dénomination commerciale Marcol 82 par la société ESSO - 15 mm²/s | | |
| ⁽⁴⁾ vendu sous la dénomination commerciale CC6004 par la société Advanced Ceramics | | |

On a pesé 20 g d'huile d'enrobage telle qu'indiquée dans le tableau ci-dessus et on a ajouté 20 g d'un solvant volatil de l'huile (un alcool absolu, par exemple). On a homogénéisé la solution par agitation mécanique, par exemple au moyen d'un barreau aimanté ou d'une pale.

On a mélangé ensuite la solution obtenue avec 80 g de nitrure de bore sous agitation permanente pour bien homogénéiser le tout.

On a maintenu l'agitation jusqu'à disparition complète du solvant volatil.

### Exemples 2-5

Différentes compositions sont préparées avec le nitrure de bore enrobé. Les proportions sont indiquées en % en poids.

| Composition | Ex. 2 | Ex. 3 | Ex. 4 | Ex. 5 |
|---|---|---|---|---|
| Extrait de racine de Cichorium intybus (Inuline) ⁽¹⁾ | 17 | | | |
| Polyacrylamide/isoparaffine en C₁₃₋₁₄/laureth-7⁽²⁾ | | 1,5 | | |
| Copolymère Acryloyldiméthyltaurate de sodium/acrylate d'hydroxyéthyle⁽³⁾ | | | 2 | |
| Alcool arachidylique/alcool béhénylique/ arachidyl-glucoside⁽⁴⁾ | | 1,5 | 1,5 | |
| Isohexadécane⁽⁵⁾ | | | 0,5 | |
| Lauryléthersulfate de sodium | | | | 12 |
| Cocobétaïne | | | | 2,2 |
| Cocamide MIPA⁽⁶⁾ | | | | 1,5 |
| Carbomer⁽⁷⁾ | | | | 0,2 |
| Diméthicone⁽⁸⁾ | | | | 2,7 |
| Niture de bore enrobé avec l'iso-hexadécane, comme décrit dans l'exemple 1. | 4 | 5 | 6 | 20 |
| (Rapport Nitrure de bore/huile) | (80/20) | (80/20) | (80/20) | (75/25) |
| Conservateur qs | | | | |
| Eau qsp | 100 | 100 | 100 | 100 |

| | | | | |
|---|---|---|---|---|
| ⁽¹⁾ vendu sous la marque commerciale Raftiline HP par la société ORAFTI | | | | |
| ⁽²⁾ vendu sous la marque commerciale Sepigel 305 par la société SEPPIC | | | | |
| ⁽³⁾ vendu sous la marque commerciale Simulgel NS par la société SEPPIC | | | | |
| ⁽⁴⁾ vendu sous la marque commerciale Montanov 202 par la société SEPPIC | | | | |
| ⁽⁵⁾ vendu sous la marque commerciale Permethyl 101A par la société BAYER | | | | |
| ⁽⁶⁾ vendu sous la marque commerciale Empilan CIS par la société HUNSTSMAN | | | | |
| ⁽⁷⁾ vendu sous la marque commerciale Carbopol 980 par la société Noveon | | | | |
| ⁽⁸⁾ vendu sous la marque commerciale DC200 FLUID 60 000 mm²/s par la société DOW CORNING | | | | |

Ces compositions appliquées sur cheveux naturels leur confèrent après 3 minutes de temps de pose et un rinçage, un excellent effet conditionneur.

## Revendications

1. Utilisation du nitrure de bore associé à au moins une huile dans une composition cosmétique, pour le conditionnement des cheveux.

2. Utilisation selon la revendication 1, **caractérisée en ce que** le nitrure de bore est enrobé par au moins une huile.

3. Utilisation selon la revendication 1 ou 2, **caractérisée en ce que** le rapport pondéral nitrure de bore/huile(s) est supérieur ou égal à 50/50.

4. Utilisation selon la revendication 3, **caractérisée en ce que** le rapport pondéral nitrure de bore/huile est supérieur ou égal à 60/40, de préférence compris entre 60/40 et 90/10.

5. Utilisation selon l'une quelconque des revendications 1 à 4, **caractérisée en ce que** le nitrure de bore présente une taille primaire moyenne en nombre comprise entre 1 et 50 *µ*m, de préférence comprise entre 5 et 30 *µ*m.

6. Utilisation selon l'une quelconque des revendications précédentes, **caractérisée en ce que** le nitrure de bore est utilisé en une quantité comprise entre 0,01 et 20 % en poids, de préférence comprise entre 0,1 et 10 % en poids par rapport au poids de la composition.

7. Utilisation selon l'une quelconque des revendications précédentes, **caractérisée en ce que** l'huile présente une viscosité dynamique comprise inférieure à 1 Pa.s à 25 °C.

8. Utilisation selon la revendication 7, **caractérisée en ce que** l'huile présente une viscosité dynamique comprise entre 10⁻³ et 0,1 Pa.s à 25 °C.

9. Utilisation selon l'une quelconque des revendications précédentes, **caractérisée en ce que** l'huile est choisie parmi les huiles végétales, les huiles minérales, les huiles de synthèse, les huiles siliconées, les esters d'acides gras.

10. Utilisation selon la revendication 9, **caractérisée en ce que** l'huile est choisie parmi l'huile d'amande douce, l'huile d'avocat, l'huile de ricin, l'huile d'olive, l'huile de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, le beurre de karité, l'huile de palme, l'huile de noyau d'abricot, l'huile de calophyllum ; l'huile de paraffine et l'huile de vaseline ; les polydécènes, le squalane, les poly(α-oléfines) comme l'isododécane ou l'isohexadécane, les huiles végétales transestérifiées et les huiles fluorées ; les composés de formule RₐCOOR_{b} dans laquelle Rₐ représente le reste d'un acide gras supérieur comportant de 6 à 29 atomes de carbone et R_{b} représente une chaîne hydrocarbonée contenant de 3 à 30 atomes de carbone, les polydiméthylsiloxanes (PDMS), les polyorganosiloxanes phénylés ; les polysiloxanes modifiés par des acides gras, des alcools gras ou des polyoxyalkylènes, les silicones fluorées, les huiles siliconées perfluorées ; les silicones linéaires ou cycliques comportant de 2 à 7 atomes de silicium.

11. Utilisation selon la revendication 10, **caractérisée en ce que** l'huile est choisie parmi l'huile d'avocat, l'isohexadécane, une paraffine liquide et les polydiméthylsiloxanes.

12. Utilisation selon l'une quelconque des revendications précédentes, **caractérisée en ce que** la ou les huile(s) est ou sont utilisée(s) en une quantité comprise entre 0,01 et 20 % en poids, de préférence comprise entre 0,1 et 10 % en poids par rapport au poids de la composition.

13. Utilisation selon l'une quelconque des revendications précédentes, dans un shampoing, un après-shampoing, un produit de fixation et/ou de maintien de la coiffure.

14. Composition cosmétique comprenant, dans un milieu aqueux cosmétiquement acceptable, du nitrure de bore et au moins une huile en un rapport pondéral nitrure de bore/huile(s) supérieur ou égal à 50/50.

15. Composition selon la revendication 14, **caractérisée en ce que** le rapport pondéral nitrure de bore/huile est supérieur ou égal à 60/40, de préférence compris entre 60/40 et 90/10.

16. Composition selon la revendication 14 ou 15, **caractérisée en ce que** le nitrure de bore est enrobé par au moins une huile.

17. Composition selon l'une quelconque des revendications 14 à 16, **caractérisée en ce que** le nitrure de bore présente une taille moyenne en nombre comprise entre 1 et 50 *µ*m, de préférence comprise entre 5 et 30 *µ*m.

18. Composition selon 14 à 17, **caractérisée en ce que** l'huile présente une viscosité dynamique inférieure à 1 Pa.s à 25 °C.

19. Composition selon la revendication 18, **caractérisé en ce que** l'huile présente une viscosité dynamique comprise entre 10⁻³ et 0,1 Pa.s à 25 °C.

20. Composition selon l'une quelconque des revendications 14 à 19, **caractérisée en ce que** l'huile est choisie parmi les huiles végétales, les huiles minérales, les huiles de synthèse, les huiles siliconées et les esters d'acide gras.

21. Composition selon la revendication 20, **caractérisée en ce que** l'huile est choisie parmi l'huile d'amande douce, l'huile d'avocat, l'huile de ricin, l'huile d'olive, l'huile de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, le beurre de karité, l'huile de palme, l'huile de noyau d'abricot, l'huile de calophyllum ; l'huile de paraffine et l'huile de vaseline ; les polydécènes, le squalane, les poly(α-oléfines) comme l'isododécane ou l'isohexadécane, les huiles végétales transestérifiées et les huiles fluorées ; les composés de formule RₐCOOR_{b} dans laquelle Rₐ représente le reste d'un acide gras supérieur comportant de 6 à 29 atomes de carbone et R_{b} représente une chaîne hydrocarbonée contenant de 3 à 30 atomes de carbone, les polydiméthylsiloxanes (PDMS), les polyorganosiloxanes phénylés ; les polysiloxanes modifiés par des acides gras, des alcools gras ou des polyoxyalkylènes, les silicones fluorées, les huiles siliconées perfluorées ; les silicones linéaires ou cycliques comportant de 2 à 7 atomes de silicium.

22. Composition selon la revendication 21, **caractérisée en ce que** l'huile est choisie parmi l'huile d'avocat, l'isohexadécane, une paraffine liquide, et les polydiméthylsiloxanes.

23. Composition selon l'une quelconque des revendications 14 à 22, **caractérisée en ce que** le nitrure de bore est présent en une quantité comprise entre 0,01 et 20 % en poids, de préférence entre 0,1 et 10 % en poids par rapport au poids de la composition.

24. Composition selon l'une quelconque des revendications 14 à 23, **caractérisée en ce que** la ou les huile(s) est ou sont présente(s) en une quantité comprise entre 0,01 et 20 % en poids, de préférence comprise entre 0,1 et 10 % en poids par rapport au poids de la composition.

25. Composition selon l'une quelconque des revendications 14 à 24, **caractérisée en ce que** le milieu aqueux cosmétiquement acceptable comprend l'eau ou un mélange d'eau et d'un solvant cosmétiquement acceptable.

26. Composition selon la revendication 25, **caractérisée en ce que** le solvant cosmétiquement acceptable est choisi parmi les alcools inférieurs en C₁-C₄, les polyols, et leurs mélanges.

27. Composition selon l'une quelconque des revendications 14 à 26, **caractérisée en ce qu'**elle comprend en outre un ou plusieurs additifs classiques choisis parmi des agents tensioactifs anioniques, cationiques, non ioniques, amphotères ou zwittérioniques, des polymères anioniques, cationiques, non-ioniques amphotères ou zwitteroniques, des filtres UV, des parfums, des agents nacrants, des épaississants naturels ou synthétiques, des alcools gras en C₁₂-C₃₀, des colorants, des conservateurs, des agents de stabilisation de pH, des vitamines, des provitamines, des agents antimicrobiens, les agents anti-oxydants ou réducteurs, et les agents acides ou alcalins.

28. Composition selon la revendication 27, **caractérisée en ce qu'**elle comprend au moins un tensioactif cationique choisi parmi les sels d'alkylamidoalkyltrialkylammonium, les dérivés d'imidazoline, ou les oxydes d'amines à caractère cationique.

29. Composition selon l'une quelconque des revendications 14 à 28, **caractérisée en ce qu'**elle se présente sous la forme d'une émulsion huile-dans-eau.

30. Procédé de traitement capillaire des cheveux consistant à appliquer sur les cheveux, une quantité efficace d'une composition telle que décrite à l'une quelconque des revendications 14 à 29, et à rincer ou non après un éventuel temps de pose.
